# EUROPÄISCHE PATENTSCHRIFT

(11) **EP 1 443 921 B1**
(45) Veröffentlichungstag und Bekanntmachung des Hinweises auf die Patenterteilung: **20.04.2005**
(21) Anmeldenummer: 02802986.6
(22) Anmeldetag: 02.11.2002
(51) Int. Cl.: A61K 31/265, A61P 31/18, A61P 31/22, A61P 35/00, A61P 37/02, A61P 43/00

(54) **HERSTELLUNG REINER STEREOISOMERE VON TRICYCLO-[5.2.1.0(2.6) ]-DEC-9-YL-XANTHOGENAT SOWIE DEREN VERWENDUNG ALS ARZNEIMITTEL**
PRODUCTION OF PURE STEREOISOMERS OF TRICYCLO-[5.2.1.0(2.6)]-DEC-9-YL-XANTHATE ESTERS AND THEIR USE AS MEDICAMENTS
PRODUCTION DES STEREOISOMERES PURS DES ESTERS DU TRICYCLO-[5.2.1.0(2.6)]-DEC-9-YL-XANTHATE ET LEURS UTILISATIONS COMME MEDICAMENTS

(30) Priorität: 17.11.2001 DE 10156617
(43) Veröffentlichungstag der Anmeldung: 11.08.2004
(73) Patentinhaber: BioSphings AG, 60329 Frankfurt (DE)
(72) Erfinder: AMTMANN, Eberhard, 69121 Heidelberg (DE)
(74) Vertreter: Wagner, Jutta, Dr.
(86) Internationale Anmeldenummer: PCT/EP2002/012242
(87) Internationale Veröffentlichungsnummer: WO 2003/041702

(56) Entgegenhaltungen:
- WO-A-96/14841
- DE-A- 10 117 728
- US-A- 4 602 037
- US-A- 4 851 435
- US-A1- 2002 177 583
- GONZALEZ-ROURA, A; CASAS, J; LLEBARIA, A: "Synthesis and phospholipase C inhibitory activity of D609 diastereoisomers" LIPIDS, Bd. 37, Nr. 4, 2002, Seiten 401-406, XP009006086

## Beschreibung

Die vorliegende Erfindung betrifft pharmazeutische Formulierungen enthaltend Exo/Exo-Tricyclo[5.2.1.0^{2.6}]-dec-9-yl-xanthogenat, Arzneimittel zur Behandlung von Herpes genitales, Herpes labiales, AIDS, Tumoren oder Autoimmunerkrankungen die Exo/Exo-Tricyclo[5.2.1.0^{2.6}]-dec-9-yl-xanthogenat als Wirkstoff enthalten sowie Verfahren zur Herstellung von reinen Stereoisomeren von Tricyclo[5.2.1.0^{2.6}]-decan-9-ol und Tricyclo[5.2.1.0^{2.6}]-dec-9-yl-xanthogenat.

Die Bekämpfung von viralen Erkrankungen bereitet erhebliche Probleme, da Vire zu ihrer Vermehrung die körpereigenen Mechanismen nutzen. Zudem sind Viren in der Lage, durch schnelle Mutation zunächst wirksame Therapeutika in sehr kurzer Zeit wirkungslos zu machen. Daher besteht ein ständiges Bedürfnis nach neuen und wirksameren Arzneimitteln gegen Viruserkrankungen.

In der US Patentschrift 4,602,037 werden Xanthogenderivate der allgemeinen Formel I: 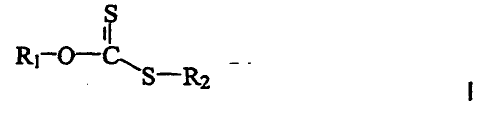 wobei R₁ für einen gegebenenfalls substituierten Aryl- oder Alkylrest steht, und ihre Verwendung als antivirale Mittel beschrieben. Als Beispiele für R₁ sind Cyclododecyl, Dodecyl, Benzyl, Cyclohexyl, Adamantyl, Tricyclo[5,2,1,0^{2,6}]-decyl, 4-Isobomylcyclohexyl und Bicyclo[2,2,1^{1,4}]-heptyl offenbart.

Die US 4,981,869 beschreibt dieselben Verbindungen wie US 4,602,037 als Antitumormittel.

Die US 4,851,435 beschreibt den Einsatz von ionischen Detergentien als wirkungsverstärkende Adjuvantien für Xanthogenderivate der Formel I.

In der WO 96/14841 wird die Inkorporierung von Xanthogenaten der Formel I und Adjuvantien in Lipid- oder Steroid-basierende Trägersubstanzen offenbart. Die Einbringung in eine Trägersubstanz soll die Verträglichkeit der Mittel verbessern.

Die nicht vorveröffentlichte DE 101 17 728 offenbart pharmazeutische Formulierungen, die ein Xanthogenat, gegebenenfalls ein die Aktivität erhöhendes Adjuvans und einen Emulgator, der die irritierende Wirkung von Xanthogenat und dem die Aktivität erhöhenden Adjuvans reduziert, enthalten. Der Emulgator entspricht stofflich der in WO 96/14841 genannten Trägersubstanz. Jedoch werden im Gegensatz zu der WO 96/14841 gemäß DE 101 17 728 Wirkstoff und Emulgator lediglich vermischt. Ein Inkorporieren des Wirkstoffs in den Träger ist entbehrlich, wodurch das Verfahren erheblich vereinfacht wird.

In all diesen Veröffentlichungen zählt Tricyclo[5.2.1.0^{2.6}] dec-9-yl-xanthogenat zu den bevorzugten Verbindungen. Tricyclo[5.2.1.0^{2.6}] dec-9-yl-xanthogenat kann in vier verschiedenen Isomeren vorliegen: Exo/Exo, Exo/Endo, Endo/Exo und Endo/Endo. In den oben genannten Veröffentlichungen wurde mit nicht genauer definierten Isomerengemischen gearbeitet. In der US Patentschrift 4,602,037 sind von Bicyclo[2,2,1^{1,4}]-heptyl sowohl exo als auch endo Isomer beschrieben und die antivirale Wirkung wird als identisch bezeichnet.

Es wurde nun überraschend gefunden, dass isomerenreines Tricyclo[5.2.1.0^{2.6}] dec-9-yl-xanthogenat eine gegenüber dem im Stand der Technik allein beschriebenen Isomerengemisch deutlich verbesserte antivirale Wirksamkeit aufweist. Das reine Exo/Exo-Isomer zeigt eine etwa 4-10 fach stärkere antivirale Wirkung als das Gemisch aus 83 % Exo/Exo-Isomer und 17 % Exo/Endo, Endo/Exo und Endo/ Endo-Isomer. Dieses Gemisch wird bei der Umsetzung des kommerziell erhältlichen Tricyclo[5.2.1.0^{2.6}] decan-9-ol mit Alkalimetall und CS₂ in inerten Lösungsmitteln erhalten.

Die vorliegende Erfindung betrifft daher pharmazeutische Formulierungen und Arzneimittel daraus, die reines Exo/Exo-Tricyclo[5.2.1.0^{2.6}] dec-9-yl-xanthogenat als Wirkstoff enthalten. Die erfindungsgemäßen Arzneimittel eignen sich zur Behandlung von Virus-, Tumor- und Autoimmunerkrankungen. Als reines Isomer werden hier solche Verbindungen bezeichnet, die neben dem benannten Isomer nicht mehr als 5%, vorzugsweise nicht mehr als 1 % und insbesondere weniger als 0,1 % der anderen möglichen Isomeren enthalten.

Es hat sich überraschenderweise gezeigt, dass sich die Exo/Exo, Exo/Endo, Endo/Exo und Endo/Endo-Isomere von Tricyclo[5.2.1.0^{2.6}] decan-9-ol durch fraktionierte Destillation trennen lassen und die reinen Isomere auch bei der Umsetzung zu Xanthogenaten erhalten bleiben.

Die Erfindung betrifft demnach auch die Herstellung reiner Isomeren von Tricyclo[5.2.1.0^{2.6}] decan-9-ol und Tricyclo[5.2.1.0^{2.6}] dec-9-yl-xanthogenat.

Die erfindungsgemäßen Arzneimittel können neben dem Wirkstoff auch übliche Träger- und Hilfsstoffe enthalten. Es können auch andere Wirkstoffe enthalten sein, soweit sie weder die Wirkung noch die Stabilität des erfindungsgemäßen Xanthogenats beeinträchtigen.

Insbesondere werden die aus der US 4,851,435 bekannten Adjuvantien, bevorzugt ionische Detergentien, besonders bevorzugt eine Fettsäure mit 6 -19 C-Atomen oder deren Salz, zugesetzt. Insbesondere bevorzugt sind die Kaliumsalze der Decan- Undecan- oder Laurinsäure. Das die Aktivität erhöhende Adjuvans kann auch ein Sulfat mit einem aliphatischen Rest von 8-18 C-Atomen sein. Besonders bevorzugt ist Na-Laurinsulfat. Weiter kommt für das Adjuvans Deoxycholinsäure oder ein pharmazeutisch verträgliches Salz davon oder eine Phosphonsäure in Betracht.

Weiterhin können Wirkstoff und gegebenenfalls Adjuvans entsprechend der WO 96/14841 in Lipid- oder Steroid-basierende Trägersubstanzen, insbesondere in ein Steroid wie Cholesterin, Cholestanol, Cholansäure, Chondrillasterol und α, β, γ Sisterol, inkorporiert sein. Insbesondere werden Wirkstoff und gegebenenfalls Adjuvans entsprechend der DE 101 17 728 mit der Trägersubstanz vermischt. Besonders bevorzugt ist Cholesterin. Als Trägersubstanz eignen sich auch Phospholipide, insbesondere Phosphatidylcholin, Phosphatidylserin, Phosphatidylinositol oder Stearylamin.

Besonders bevorzugt ist eine Formulierung enthaltend Exo/Exo-Tricyclo[5.2.1.0^{2.6}] dec-9-yl-xanthogenat mit dem Na- oder K-Salz der Decansäure als die Aktivität erhöhendem Adjuvans und Cholesterin. Insbesondere kommen auf einen Teil Xanthogenat ein Teil Kaliumsalz der Decansäure und 4 Teile Cholesterin.

Eine weitere bevorzugte Formulierung enthält Exo/Exo-Tricyclo[5.2.1.0^{2.6}] dec-9-yl-xanthogenat, Phosphatidylcholin und als die Aktivität erhöhendes Adjuvans das Na- oder K-Salz der Decansäure. Insbesondere kommen auf einen Teil Xanthogenat ein Teil Decansäure und 4 Teile Phosphatidylcholin.

Die erfindungsgemäßen Arzneimittel können als orale, parenterale und topische Darreichungsformen sowie als Injektionslösungen formuliert werden. Bevorzugt ist die topische Anwendung in Form von Salben, wobei als Salbengrundlage eine lipophile Substanz benutzt wird. Vorzugsweise wird als Salbengrundlage Vaseline verwendet.

Die Dosierung richtet sich nach Schwere und Verlauf der Erkrankung, wobei durch die höhere Wirksamkeit des erfindungsgemäßen isomerenreinen Xanthogenats die Dosis gegenüber der Verabreichung des Isomerengemisches verringert werden kann. Typischerweise werden Salben mit Konzentrationen von 0,5 bis 10 %, insbesondere 1 bis 5 % Xanthogenat mehrmals täglich auf die zu behandelnde Hautfläche aufgetragen.

Die folgenden Beispiele erläutern die Erfindung weiter, ohne sie jedoch zu beschränken.

### Beispiel 1: Herstellung von Exo/Exo Tricyclo[5.2.1.0^{2.6}] decan-9-ol

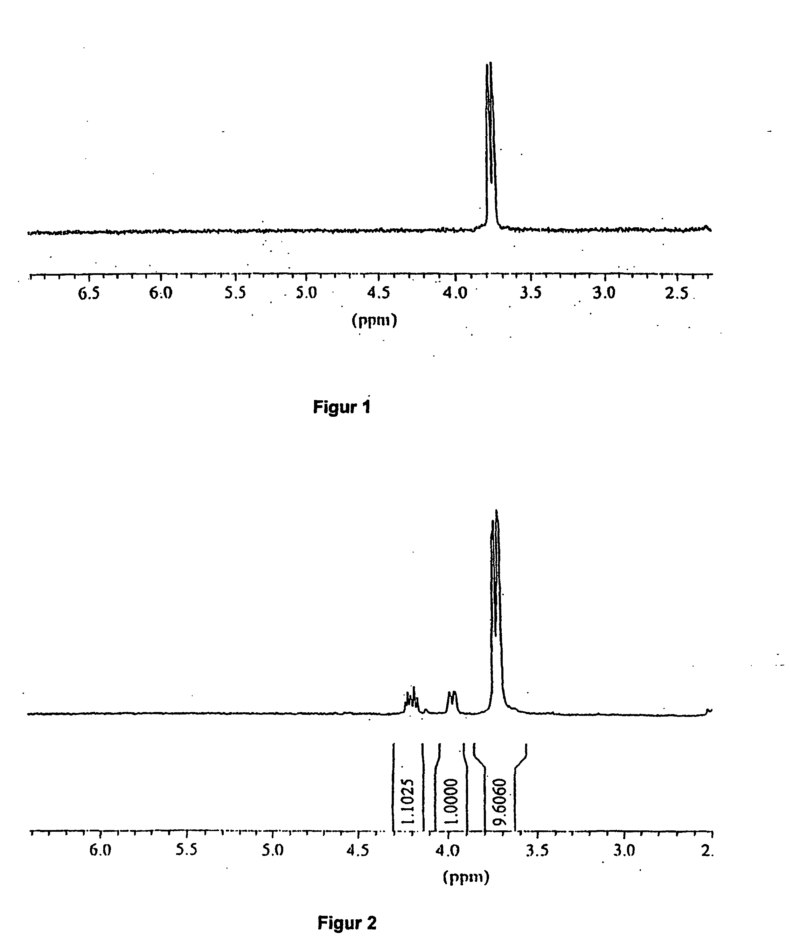

Ein kommerziell erhältliches Gemisch von Isomeren von Tricyclo[5.2.1.0^{2.6}] decan-9-ol wurde bei 49 °C im Vakuum (10⁻⁴ Torr) destilliert. Als erste Fraktion wurde das reine Exo/Exo-Isomer gesammelt. Die Isomeren können im NMR-Spektrum identifiziert werden. In Figur 1 ist ein Ausschnitt aus dem ¹H-NMR Spektrum der ersten Fraktion dargestellt. Es wurde mit einem Bruker DRX 250 Spektrometer in CDCl₃ Lösung mit CHCl₃ als Standard bei 250 MHz aufgenommen. Man erkennt, dass das Exo/Exo-Isomer einen Doppel-Peak zwischen 3,7 und 3,8 ppm zeigt. In Figur 2 ist das entsprechende Spektrum des Isomerengemisches gezeigt. Die anderen Isomeren zeigen Peaks bei ca. 4.0 ppm und zwischen 4,1 und 4,3 ppm.

### Beispiel 2: Herstellung von Exo/Exo-Tricyclo[5.2.1.0^{2.6}] dec-9-yl-xanthogenat

457 g (ca. 3 mol) Exo/Exo-Tricyclo[5.2.1.0^{2.6}] decan-9-ol wurden in einer Schutzgasatmosphäre (Stickstoff) bei 150 bis 160 °C portionsweise mit insgesamt 19,55 g entkrustetem Kalium unter Rühren vermengt. Die Mischung wurde bei dieser Temperatur gehalten bis das Metall komplett umgesetzt war Anschließend wurde überschüssiger Alkohol im Vakuum abdestilliert. Das Alkoholat wurde im Vakuum getrocknet und in 500 ml Tetrahydrofuran gelöst. Unter Kühlung wurden 31 ml (0,5 mol) CS₂ in 150 ml Ether nach und nach zugefügt. Die Mischung wurde 1 h bei 40 °C gerührt. Das Xanthogenat wurde durch Zugabe von 1 l trockenem Ether ausgefällt, mit der Nutsche abfiltriert und auf dem Filter gründlich mit Ether gewaschen. Nach der Umkristallisation aus Ethanol wurden 104 g (78 % d.Th.) feine gelbe Nadeln erhalten. Die Reinheit wurde durch NMR-Spektroskopie nachgewiesen.

### Beispiel 3:

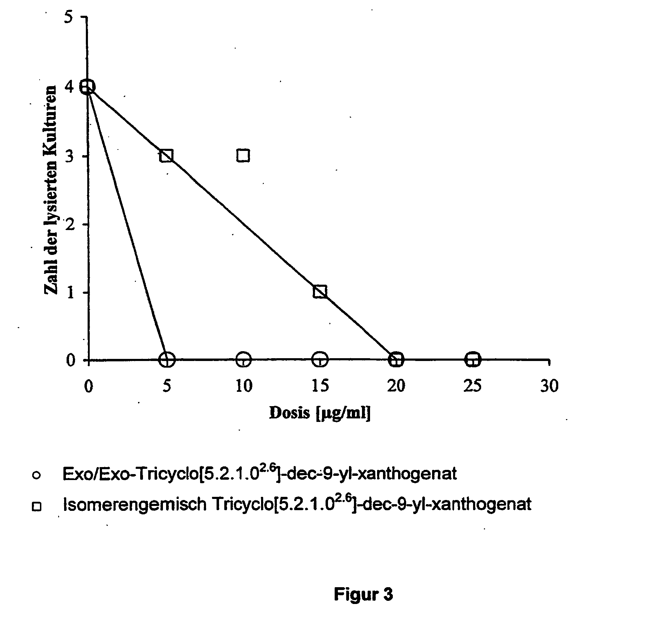

Die Hemmung der Herpesvermehrung durch Exo/Exo-Tricyclo[5.2.1.0^{2.6}] dec-9-ylxanthogenat wurde mittels Messung des zytopathogenen Effektes ermittelt. Dazu wurden Affennierenzellen (Stamm Rita) in Linbro-Platten ausgesät. Nach 24 h wurden zu jeder Kultur 50 plaquebildende Einheiten Herpes simplex Virus 1 gegeben. Nach einer Stunde Adsorption wurde frisches Kulturmedium, das auf pH 6,8 eingestellt war, zugegeben. Jeweils 4 Kulturen wurden mit entweder reinem Exo/Exo-Isomer oder einem Isomerengemisch, das 83 % Exo/Exo-Isomer enthielt, in folgenden Konzentrationen behandelt: 0, 5, 10, 15 oder 20 µg/ml. Nach sechs Tagen wurden die Kulturen mikroskopisch ausgewertet. Das Ergebnis ist in Figur 3 dargestellt. Unbehandelte Kulturen waren auf Grund der Virusvermehrung vollkommen lysiert (zytopathogener Effekt). Alle Kulturen, die mit dem Exo/Exo-Isomer behandelt worden waren wiesen keinen zytopathogener Effekt auf. Das heißt die Virusvermehrung war bereits bei einer Konzentration von 5 µg/ml vollständig unterdrückt. Das Isomerengemisch zeigte diesen Effekt erst bei einer Konzentration von 20 µg/ml.

### Beispiel 4:

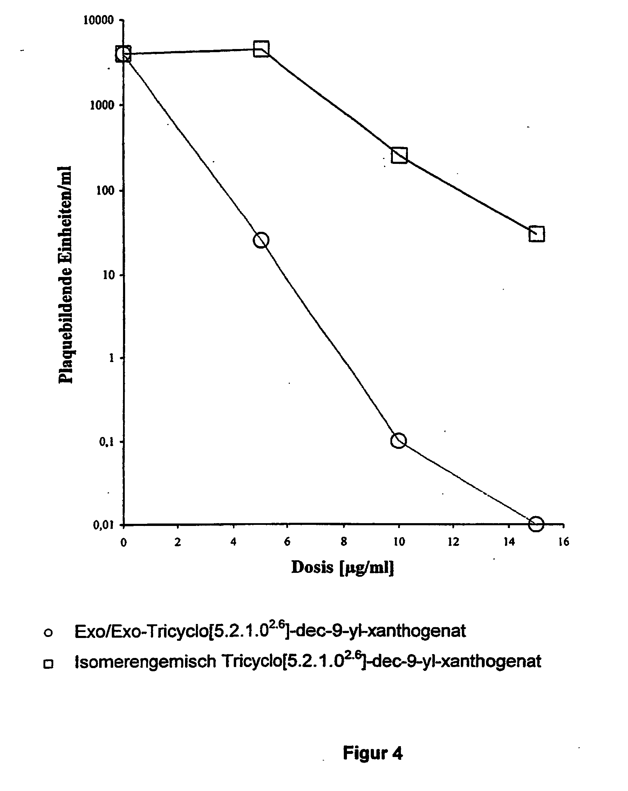

Die Hemmung der Herpesvermehrung durch Exo/Exo-Tricyclo[5.2.1.0^{2.6}] dec-9-ylxanthogenat wurde durch Messung der Virusvermehrung ermittelt. Dazu wurden Affennierenzellen (Stamm Rita) in Linbro-Platten ausgesät. Nach 24 h wurden zu jeder Kultur 100 plaquebildende Einheiten Herpes simplex Virus 1 gegeben. Nach einer Stunde Adsorption wurde frisches Kulturmedium, das auf pH 6,8 eingestellt war zugegeben. Jeweils 4 Kulturen wurden mit entweder reinem Exo/Exo-Isomer oder einem Isomerengemisch, das 83 % Exo/Exo-Isomer enthielt in folgenden Konzentrationen behandelt: 0, 5, 10 oder 15 µg/ml. Nach vier Tagen wurde die Viruskonzentration in den Überständen der Kulturen durch Plaqueassays bestimmt. Die Mittelwerte wurden für jede Konzentration berechnet und sind in Figur 4 in eine Dosis-Wirkungskurve eingetragen. Aus den Kurven wurde die Konzentration, bei der die Virusvermehrung um 50% gehemmt war (IC50), bestimmt. Für das Exo/Exo-Isomer wurde eine IC50 von 0,6 µg/ml gefunden. Für das Isomerengemisch wurde eine IC50 von 7,6 gefunden. Das heißt die Virusvermehrung wurde durch das reine Exo/Exo-Isomer bei einer mehr als 10-fach niedrigeren Konzentration als durch das Isomerengemisch um die Hälfte reduziert.

## Patentansprüche

1. Pharmazeutische Formulierung enthaltend ein Xanthogenat der Formel I 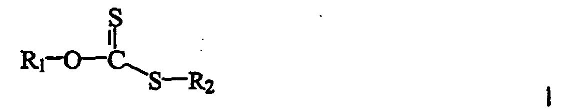 wobei R₁ für Tricyclo[5.2.1.0^{2.6}]-decyl und R₂ für ein Metallatom, eine gegebenenfalls substituierte Alkyl-, Alkoxy-, Amino- oder Ammoniumgruppe oder Halogen steht, **dadurch gekennzeichnet, dass** es sich um das reine Exo/Exo- Tricyclo[5.2.1.0^{2.6}]-dec-9-yl-xanthogenat handelt.

2. Pharmazeutische Formulierung nach Anspruch 1, **dadurch gekennzeichnet, dass** R₂ ein Natrium- oder Kaliumatom oder eine Dimethylglycylester- oder Methylestergruppe ist.

3. Pharmazeutische Formulierung nach Anspruch 1 oder 2, **dadurch gekennzeichnet, dass** ein Steroid oder ein Phospholipid zur Verminderung der Reizwirkung des Xanthogenats enthalten ist.

4. Pharmazeutische Formulierung nach Anspruch 3, **dadurch gekennzeichnet, dass** Cholesterin oder Phosphatidylcholin enthalten ist.

5. Pharmazeutische Formulierung nach Anspruch 3 oder 4, **dadurch gekennzeichnet, dass** je ein Teil Xanthogenat 1 bis 10, vorzugsweise 2 bis 4 Teile Steroid oder Phospholipid enthalten sind.

6. Pharmazeutische Formulierung nach einem der Ansprüche 1 bis 5, **dadurch gekennzeichnet, dass** ein ionisches Detergens, vorzugsweise eine Fettsäure mit 6 bis 19 C-Atomen oder ein Alkylsulfat mit 8 bis 18 C-Atomen, als ein Adjuvans zur Erhöhung der Aktivität des Xanthogenats enthalten ist.

7. Pharmazeutische Formulierung nach einem der Ansprüche 1 bis 5, **dadurch gekennzeichnet, dass** Deoxycholinsäure oder ein pharmazeutisch verträgliches Salz davon als Adjuvans zur Erhöhung der Aktivität des Xanthogenats enthalten ist

8. Pharmazeutische Formulierung nach einem der Ansprüche 1 bis 5, **dadurch gekennzeichnet, dass** eine Phosphonsäure als Adjuvans zur Erhöhung der Aktivität des Xanthogenats enthalten ist

9. Verfahren zur Herstellung reiner Stereoisomere von Tricyclo[5.2.1.0^{2.6}] decan-9-ol, **dadurch gekennzeichnet, dass** ein Gemisch der Stereoisomeren bei vermindertem Druck einer fraktionierten Destillation unterworfen wird.

10. Verfahren zur Herstellung reiner Stereoisomere von Tricyclo[5.2.1.0^{2.6}] dec-9-ylxanthogenaten, wobei ein isomerenreines Tricyclo[5.2.1.0^{2.6}] decan-9-ol mit einem Alkalimetall oder Metallhydrid zum Alkoholat umgesetzt und daraus nach Lösen in einem inerten Lösungsmittel durch Zugabe von CS₂ das Xanthogenat dargestellt wird.

11. Arzneimittel zur Behandlung von Herpes genitales, Herpes labiales, AIDS, Tumoren oder Autoimmunerkrankungen, **dadurch gekennzeichnet, dass** es eine pharmazeutische Formulierung gemäß einem der Ansprüche 1 bis 8 als Wirkstoff enthält.

12. Arzneimittel gemäß Anspruch 11, **dadurch gekennzeichnet, dass** es eine Salbe oder eine Creme ist

13. Verwendung von reinem Exo/Exo Tricyclo[5.2.1.0^{2.6}] dec 9-yl-xanthogenat zur Herstellung von Arzneimitteln zur Behandlung von Herpes genitales, Herpes labiales, AIDS, Tumoren oder Autoimmunerkrankungen.

## Claims

1. Pharmaceutical formulation containing a xanthogenate corresponding to formula I 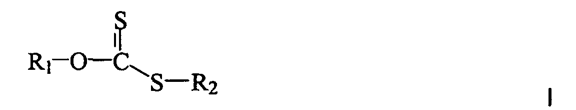 whereby R₁ corresponds to tricyclo[5.2.1.0^{2.6}]-decyl and R₂ corresponds to a metal atom, a possibly substituted alkyl, alkoxy, amino or ammonium group or halogen, **characterized in that** it is pure exo/exo-tricyclo[5.2.1.0^{2.6}]-dec-9-yl-xanthogenate.

2. Pharmaceutical formulation according to Claim1, **characterized in that** R₂ is a sodium or potassium atom or a dimethylglycylester or methylester group.

3. Pharmaceutical formulation according to Claim 1 or 2, **characterized in that** it contains a steroid or a phospholipid to reduce the irritant effect of the xanthogenate.

4. Pharmaceutical formulation according to Claim 3, **characterized in that** it contains cholesterol or phosphatidylcholine.

5. Pharmaceutical formulation according to Claim 3 or 4, **characterized in that** it contains 1-10 parts, preferably 2 to 4 parts, steroid or phospholipd per one part xanthogenate.

6. Pharmaceutical formulation according to any one of the Claims 1 to 5, **characterized in that** it contains an ionic detergent, preferably a fatty acid with 6 to 19 carbon atoms or an alkyl sulfate with 8 to 18 carbon atoms, as an adjuvant to enhance the activity of the xanthogenate.

7. Pharmaceutical formulation according to any one of the Claims 1 to 5, **characterized in that** it contains deoxycholic acid or a pharmaceutically tolerable salt thereof as adjuvant to enhance the activity of the xanthogenate.

8. Pharmaceutical formulation according to any one of the Claims 1 to 5, **characterized in that** it contains a phosphonic acid as adjuvant to enhance the activity of the xanthogenate.

9. Method for the production of pure stereoisomers of tricyclo[5.2.1.0^{2.6}] decan-9-ol, **characterized in that** a mixture of stereoisomers is subjected to fractional distillation under reduced pressure.

10. Method for the production of pure stereoisomers of tricyclo[5.2.1.0^{2.6}] dec-9-yl-xanthogenates, whereby an isomerically pure tricyclo[5.2.1.0^{2.6}] decan-9-ol is reacted with an alkali metal or metal hydride to generate the alcoholate which is then dissolved in an inert solvent and used to prepare the xanthogenate by adding CS₂.

11. Drug for the treatment of genital herpes, labial herpes, AIDS, tumors or autoimmune diseases, **characterized in that** it contains a pharmaceutical formulation according to any one of the Claims 1 to 8 as active ingredient.

12. Drug according to Claim 11, **characterized in that** it is an ointment or cream.

13. Use of pure exo/exo-tricyclo[5.2.1.0^{2.6}] dec-9-yl-xanthogenate for the manufacture of drugs for the treatment of genital herpes, labial herpes, AIDS, tumors or autoimmune diseases.

## Revendications

1. Formulation pharmaceutique contenant un xanthogénate de formule I 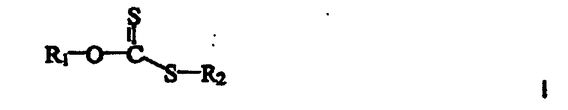 dans laquelle R₁ représente un groupe tricyclo[5.2.1.0^{2.6}]-décyle et R₂ un atome métallique, un groupe alkyle, alcoxy, amino ou ammonium éventuellement substitué, ou un atome d'halogène, **caractérisé en ce qu'**il s'agit de l'exo/exo-tricyclo[5.2.1.0^{2.6}]-déc-9-yl-xanthogénate pur.

2. Formulation pharmaceutique selon la revendication 1, **caractérisée en ce que** R₂ représente un atome de sodium ou de potassium ou un groupe diméthylglycylester ou méthylester.

3. Formulation pharmaceutique selon la revendication 1 ou 2, **caractérisée en ce qu'**elle contient un stéroïde ou un phospholipide destiné à diminuer l'action irritante du xanthogénate.

4. Formulation pharmaceutique selon la revendication 3, **caractérisée en ce qu'**elle contient du cholestérol ou de la phosphatidylcholine.

5. Formulation pharmaceutique selon la revendication 3 ou 4, **caractérisée en ce que**, pour une partie de xanthogénate, elle contient de 1 à 10, de préférence de 2 à 4 parties de stéroïde ou de phospholipide.

6. Formulation pharmaceutique selon l'une quelconque des revendications 1 à 5, **caractérisée en ce qu'**elle contient un détergent ionique, de préférence un acide gras comprenant 6 à 19 atomes de C ou un alkylsulfate comprenant 8 à 18 atomes de C, comme adjuvant destiné à élever l'activité du xanthogénate.

7. Formulation pharmaceutique selon l'une quelconque des revendications 1 à 5, **caractérisée en ce qu'**elle contient de l'acide désoxycholinique ou un sel de celui-ci pharmaceutiquement compatible, comme adjuvant destiné à élever l'activité du xanthogénate.

8. Formulation pharmaceutique selon l'une quelconque des revendications 1 à 5, **caractérisée en ce qu'**elle contient un acide phosphonique, comme adjuvant destiné à élever l'activité du xanthogénate.

9. Procédé de préparation des stéréo-isomères purs du tricyclo[5.2.1.0^{2.6}]-décane-9-ol, **caractérisé en ce qu'**on soumet un mélange de stéréo-isomères à une distillation fractionnée sous pression réduite.

10. Procédé de préparation de stéréo-isomères purs de tricyclo[5.2.1.0^{2.6}]-déc-9-yl-xanthogénates, dans lequel on fait réagir un isomère pur de tricyclo[5.2.1.0^{2.6}]-décane-9-ol avec un métal alcalin ou un hydrure de métal pour former un alcoolate et après dissolution de ce dernier dans un solvant inerte, on prépare le xanthogénate par addition de CS₂.

11. Médicament destiné au traitement de l'herpès génital, de l'herpès labial, du SIDA, de tumeurs ou de maladies auto-immunes, **caractérisé en ce qu'**il contient, en tant que principe actif, une formulation pharmaceutique selon l'une quelconque des revendications 1 à 8.

12. Médicament selon la revendication 11, **caractérisé en ce qu'**il se présente sous forme d'onguent ou de crème.

13. Utilisation de l'exo/exo-tricyclo[5.2.1.0^{2.6}]-déc-9-yl-xanthogénate pur pour la préparation de médicaments destinés au traitement de l'herpès génital, de l'herpès labial, du SIDA, de tumeurs ou de maladies auto-immunes.
